# EUROPEAN PATENT SPECIFICATION

(11) **EP 2 889 024 B1**
(45) Date of publication and mention of the grant of the patent: **19.09.2018**
(21) Application number: 12883565.9
(22) Date of filing: 27.08.2012
(51) Int. Cl.: A61J 1/05, A61J 1/10, A61M 1/02

(54) **BLOOD BAG AND BLOOD BAG SYSTEM PROVIDED THEREWITH**
BLUTBEUTEL UND BLUTBEUTELSYSTEM DAMIT
POCHE DE SANG ET SYSTÈME DE POCHE DE SANG LA COMPRENANT

(43) Date of publication of application: 01.07.2015
(73) Proprietor: Terumo Kabushiki Kaisha, Tokyo 151-0072 (JP)
(72) Inventor: HAYAKAWA, Aya, Ashigarakami-gun Kanagawa 259-0151 (JP); AKIYAMA, Masahiro, Ashigarakami-gun Kanagawa 259-0151 (JP)
(74) Representative: Casalonga
(86) International application number: PCT/JP2012/071617
(87) International publication number: WO 2014/033819

(56) References cited:
- EP-A1- 2 080 501
- EP-A2- 0 132 632
- WO-A1-2009/022922
- WO-A1-2011/093260
- JP-A- 2008 178 554
- JP-U- S5 237 897
- JP-U- S53 133 496

## Description

### Technical Field

The present invention relates to a blood bag including a bag body formed of a soft material in a bag-shaped manner, and a blood bag system including the blood bag.

The EP132632 discloses such a compartmented flexible solution container.

### Background Art

In recent years, component blood transfusion, in which blood transfusion of only a component necessary for a patient is performed, has been widely performed. When the component blood transfusion is performed, a blood bag system for dividing blood (whole blood) collected from a donor into a plurality of blood components, and housing and storing the plurality of blood components is used.

This sort of blood bag system includes an initial flow blood bag (blood bag) for housing a predetermined amount of blood (initial flow blood) that flows out immediately after a blood-collecting needle is inserted into the donor, and a blood-collecting bag for housing blood that flows out after the initial flow blood flows.

In the initial flow blood, a piece of skin, skin indigenous bacteria, and the like may be mixed. However, according to such a blood bag system, the initial flow blood can be reliably removed from blood for blood transfusion.

Then, the initial flow blood housed in the initial flow blood bag is transferred to a blood sampling tube through a blood-collecting holder connected to the initial flow blood bag, and is provided as testing blood for blood transfusion, and thus a necessary collection amount of the initial flow blood is determined.

Therefore, typically, a mark that indicates that the necessary collection amount of the initial flow blood has been housed is put on the initial flow blood bag, and when the liquid level of the initial flow blood reaches the mark, inflow of the initial flow blood to the initial flow blood bag is stopped.

However, when the initial flow blood bag formed of a soft material in a bag-shaped manner is used, air in a tube that connects the blood-collecting needle and the initial flow blood bag flows into the initial flow blood bag in collecting the initial flow blood, and thus the initial flow blood bag may be inflated by the air (for example, see JP 2008-178553 A). The amount of air flows into the initial flow blood bag varies depending on the specification of the blood bag system, a sterilization method or the like, and thus it is not easy to control the air to a fixed amount.

Therefore, even if the inflow of the initial flow blood to the initial flow blood bag is stopped when the liquid level of the initial flow blood in the initial flow blood bag has reached the predetermined mark, excess/shortage of the collection amount of the initial flow blood is caused, and the necessary collection amount or more of the initial flow blood may not be able to be reliably collected.

By the way, the initial flow blood collected in the initial flow blood bag is transferred to the blood sampling tube through the blood sampling holder connected to the initial flow blood bag. In this case, typically, an operator performs transfer work in a state of bending over so as not to allow the air in the initial flow blood bag to flow into the blood sampling tube, and thus the transfer work is a considerable burden on the operator.

As an initial flow blood bag that prevents the inflow of the air in the initial flow blood bag to the blood sampling tube and reduces the burden of the transfer work of the initial flow blood to the blood sampling tube, a one in which a bag-shaped bag body is partitioned into a blood-housing portion and an air-housing portion with a partition wall (partition), and a blood inflow portion and a blood outflow portion are provided in the blood-housing portion provided is known (for example, see JP 2008-110043 A, JP 2008-131962 A, and WO 2010/041603 A pamphlet).

### Summary of Invention

However, in the conventional technology such as JP 2008-110043 A, or the like, air exists in the blood-housing portion in a state where a necessary collection amount of the initial flow blood is housed in the blood-housing portion. Therefore, the degree of inflation of the blood-housing portion is changed depending on the amount of air, and variation may be caused in the collection amount of the initial flow blood.

The present invention as further disclosed in claim 1, has been made in view of the problem, and an objective is to provide a blood bag and a blood bag system including the blood bag, which can collect a fixed amount of blood to the blood bag regardless of the amount of air that has flown into the blood bag, and can reduce a burden of an operator and can suppress inflow of the air to a blood sampling tube when the blood in the blood bag is transferred to the blood sampling tube.
[1] A blood bag according to the present invention is a blood bag including a bag body formed of a soft material in a bag-shaped manner, and being able to house blood, and the bag body includes: a first housing portion provided with a blood inflow portion; a second housing portion provided with a blood outflow portion; and a communication path configured from a partition that partitions an inside of the bag body into the first housing portion and the second housing portion, and allowing the first housing portion and the second housing portion to communicate with each other.
   According to the blood bag of the present invention, the communication path is configured from the partition that partitions an inside of the bag body into the first housing portion provided with the blood inflow portion and the second housing portion provided with the blood outflow portion. Therefore, air in the second housing portion is discharged to the first housing portion, and only blood can be housed in the second housing portion, and an influence of inflation of the first housing portion by the air on inflation of the second housing portion can be made small. Accordingly, a fixed amount of blood can be collected to the blood bag.
   Further, by use of such a blood bag, not only when the blood bag is used in hanging down arrangement, but also when the blood bag is used in horizontal arrangement, the second housing portion is arranged vertically below the first housing portion during collection of blood, whereby the air in the second housing portion is discharged to the first housing portion, and only the blood can be housed in the second housing portion. Therefore, a fixed amount of blood can be collected to the blood bag.
   Further, a portion of the communication path in the bag body is bent in a state where the air in the second housing portion is discharged to the first housing portion, whereby a flow path of the communication path is blocked and inflow of the air to the second housing portion can be prevented. Accordingly, even if a blood sampling holder and a blood sampling tube connected to the blood outflow portion are arranged at positions where an operator can easily perform an operation, the air cannot flow into the blood sampling tube. Therefore, a burden of the operator can be reduced, and the inflow of the air to the blood sampling tube can be suppressed.
[2] In the blood bag, an opening of the blood inflow portion may face an opening in the communication path at a side of the first housing portion.
   According to the configuration, the opening of the blood inflow portion faces the opening in the communication path at the side of the first housing portion. Therefore, the blood flowing in from the blood inflow portion can be easily guided to the communication path.
[3] In the blood bag, a pair of the partitions may be provided to face each other along a width direction of the communication path.
   According to the configuration, the pair of partitions is provided to face each other along the width direction of the communication path. Therefore, inflow of the blood housed in the second housing portion to the first housing portion can be favorably suppressed by the pair of partitions.
[4] In the blood bag, the bag body may be configured such that soft sheet materials are layered, and a sealed portion in a periphery of the seat materials is sealed or glued, and the sealed portion may include a first sealed part in a periphery of the first housing portion, and a second sealed part in a periphery of the second housing portion, and formed independently of the first sealed part.
   According to the configuration, the first sealed part in the periphery of the first housing portion and the second sealed part in the periphery of the second housing portion are independently formed. Therefore, an influence of inflation of the first housing portion by air on inflation of the second housing portion can be made smaller.
[5] In the blood bag, an external dimension along a width direction of the second sealed part may be set smaller than an external dimension along a width direction of the first sealed part.
   According to the configuration, the external dimension of the width direction of the second sealed part is set smaller than the external dimension of the width direction of the first sealed part. Therefore, the second housing portion and the second sealed part can be easily held by fingers of a person. Accordingly, positions of the blood sampling holder and the blood sampling tube can be easily adjusted.
[6] In the blood bag, the blood inflow portion may extend toward the communication path in a state of being configured in a tube-shaped manner, and an opening of the blood inflow portion may be positioned inside the first housing portion.
   According to the configuration, the blood inflow portion is configured in a tube-shaped manner and extends toward the communication path, and the opening of the blood inflow portion is positioned inside the first housing portion. Therefore, the distance between the opening and the communication path can be made short. Accordingly, the blood flowing in from the blood inflow portion can be more reliably guided to the second housing portion. That is, direct accumulation of the blood flowing in from the blood inflow portion, in the first housing portion can be suppressed.
[7] In the blood bag, an inclined portion close to the communication path toward the first housing portion may be formed on a wall surface of the partition, the wall surface configuring the second housing portion.
   According to the configuration, the inclined portion formed on the wall surface of the partition, the wall surface configuring the second housing portion, is close to the communication path toward the first housing portion. Therefore, the air in the second housing portion can be smoothly guided to the first housing portion through the inclined portion.
[8] In the blood bag, a flat portion continuing to a side wall surface that configures the first housing portion, in an approximately right angle manner, may be formed on a wall surface of the partition, the wall surface configuring the first housing portion.
   According to the configuration, the flat portion formed on the wall surface of the partition, the wall surface configuring the first housing portion, continues to the side wall surface that configures the first housing portion, in a right angle manner. Therefore, inflow of the air in the first housing portion to the second housing portion through the communication path can be favorably suppressed.
[9] In the blood bag, a display means with which an amount of blood housed in the bag body is able to be confirmed may be attached to a wall portion that configures the communication path.
   According to the configuration, the display means with which the amount of blood housed in the bag body can be confirmed is attached to the wall surface that configures the communication path. Therefore, the collection amount of the blood in the blood bag can be easily confirmed, and adjustment of the collection amount of the blood can be easily performed.
[10] In the blood bag, the partition may extend along a width direction of the bag body in a state of being positioned in an approximately center of the bag body in the width direction, and a pair of the communication paths may be provided at both end sides of the bag body.
   According to the configuration, the partition extends along the width direction of the bag body in a state of being positioned in the approximately center of the bag body in the width direction. Therefore, an influence of inflation of the first housing portion by air on inflation of the second housing portion can be made smaller. Further, the pair of communication paths is provided at both end sides of the partition. Therefore, the blood flowing in from the blood inflow portion can be smoothly guided to the second housing portion, compared with a case where the communication path is provided at only one end portion side of the partition.
[11] In the blood bag, the blood housed in the bag body may be initial flow blood.
   According to the configuration, a fixed amount of the initial flow blood can be collected to the blood bag.
[12] In the blood bag, all air in the blood bag may be housed in the first housing portion, and only the blood may be housed in the second housing portion.
   According to the configuration, all air in the blood bag is housed in the first housing portion, and only the blood is housed in the second housing portion. Therefore, regardless of the amount of air that has flown into the blood bag, a fixed amount of blood can be reliably collected to the blood bag. Further, when the blood in the blood bag is transferred to the blood sampling tube, inflow of the air to the blood sampling tube can be reliably suppressed.
[13] A blood bag system according to the present invention is a blood bag system including: a blood bag for housing initial flow blood; a blood-collecting bag for housing blood after the initial flow blood is removed; and a separation treatment portion configured to divide the blood housed in the blood-collecting bag into a plurality of blood components, and to house the respective components in different bags, and the blood bag is the above-described blood bag.
   The blood bag system according to the present invention exhibits a similar effect to the above-described blood bag.

### Brief Description of Drawings

Fig. 1 is a plan view of a blood bag system according to an embodiment of the present invention.
Fig. 2A is an enlarged plan view of an initial flow blood bag illustrated in Fig. 1, and Fig. 2B is an enlarged plan view illustrating a state in which initial flow blood is collected in the initial flow blood bag.
Fig. 3A is a partially omitted perspective view illustrating a state in which the initial flow blood bag is sandwiched by fingers and is bent, when the initial flow blood in the initial flow blood bag is transferred to a blood sampling tube, and Fig. 3B is a partially omitted perspective view illustrating a state in which the initial flow blood bag is bent in an acute angle, when the initial flow blood in the initial flow blood bag is transferred to the blood sampling tube.
Fig. 4A is a plan view of an initial flow blood bag according to a first modification, Fig. 4B is a plan view of an initial flow blood bag according to a second modification, and Fig. 4C is a plan view of an initial flow blood bag according to a third modification.
Fig. 5A is a plan view of an initial flow blood bag according to a fourth modification, and Fig. 5B is a plan view of an initial flow blood bag according to a fifth modification.

### Description of Embodiments

Hereinafter, favorable embodiments with respect to a blood bag and a blood bag system according to the present invention will be described with reference to the appended drawings.

A blood bag system 12 is used to centrifuge blood that contains a plurality of components into a plurality of components having different relative density (for example, three components of a low relative density component, a medium relative density component, and a large relative density component, or two components of a low relative density component and a large relative density component), and to house and store the respective components in different bags.

The blood bag system 12 according to the present embodiment is configured to centrifuge a residual blood component, which is obtained such that white blood cells and blood platelets are removed from whole blood, into two components of blood plasma and packed red blood cells, and to house and store the blood plasma and the packed red blood cells in different bags.

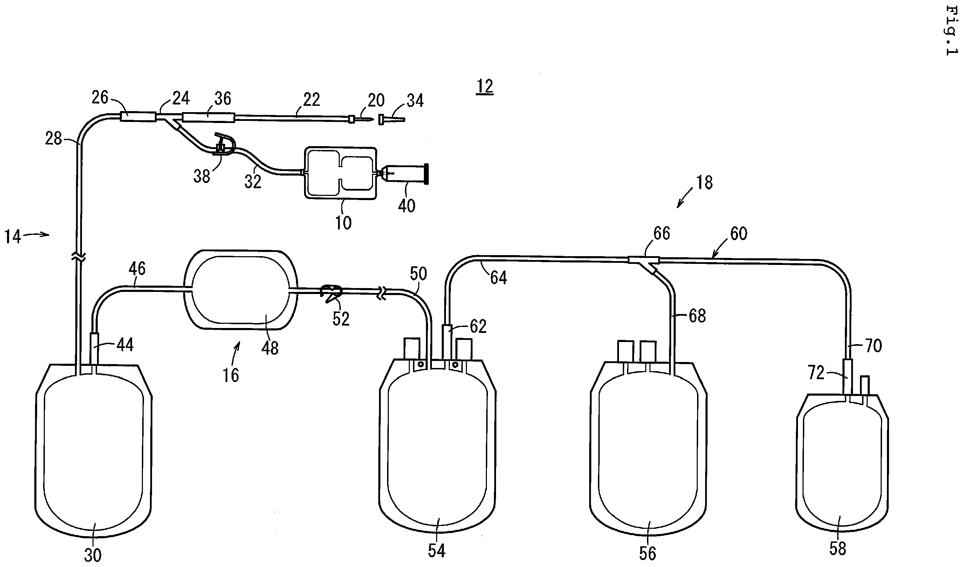

As illustrated in Fig. 1, the blood bag system 12 includes a blood-collecting portion 14 that collects blood (whole blood) from a donor, a pre-treatment portion 16 that removes predetermined blood components from the whole blood, and a separation treatment portion 18 that centrifuges the residual blood component from which the predetermined components have been removed to divide it into a plurality of blood components, and houses (accumulates) the respective components in different bags.

The blood-collecting portion 14 includes a blood-collecting needle 20, a first blood-collecting tube 22, one end of which is connected to the blood-collecting needle 20, a Y-shaped branch connector 24 connected to the other end of the first blood-collecting tube 22, a second blood-collecting tube 28 connected to the branch connector 24 through a sealing member (breaking/communication member) 26, a parent bag (blood-collecting bag) 30 connected to the other end of the second blood-collecting tube 28, a branch tube 32 communicating into the first blood-collecting tube 22 through the branch connector 24, and an initial flow blood bag (blood bag) 10 connected to the other end of the branch tube 32, for housing (accumulating) a predetermined amount of blood (initial flow blood) that flows out immediately after the blood-collecting needle 20 is inserted into the donor.

A cap 34 is mounted on the blood-collecting needle 20 before use, and a needle guard 36 is mounted after use.
The needle guard 36 is disposed movable along a longitudinal direction of the first blood-collecting tube 22. The sealing member 26 is configured such that a flow path is blocked in an initial state, but the flow path is opened by a breaking operation.

In a middle portion of the branch tube 32, a clamp 38 that blocks and releases the flow path of the branch tube 32 is provided. The clamp 38 is configured not to be opened once closed. As such a clamp 38, one similar to the clamp disclosed in JP 5-23792 B can be used, for example.

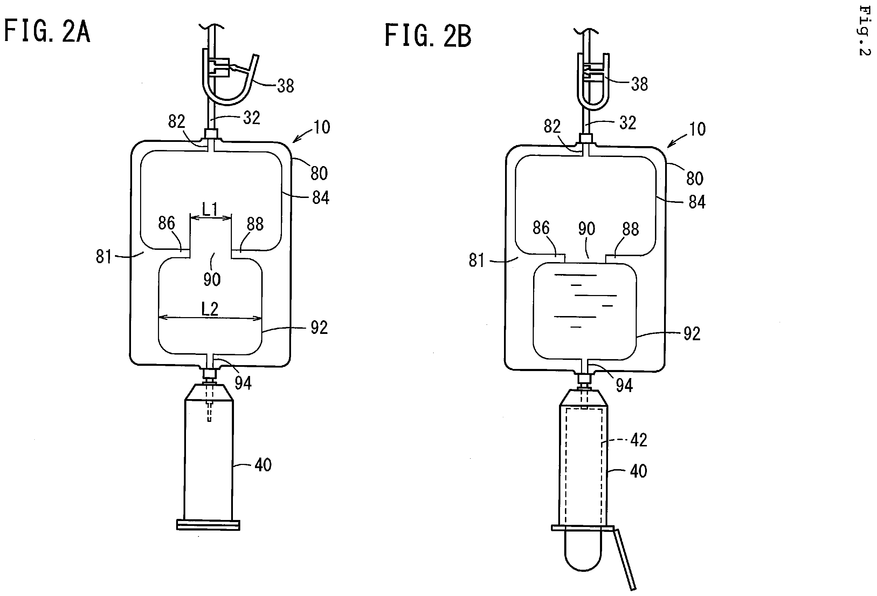

A blood sampling holder 40 is connected to the initial flow blood bag 10. A blood sampling tube 42 (see Fig. 2B) is mounted to the blood sampling holder 40, so that the initial flow blood in the initial flow blood bag 10 is transferred to the blood sampling tube 42 in a depressurized state (vacuum state). Detailed description of the initial flow blood bag 10 will be given below.

The pre-treatment portion 16 includes an inlet-side tube 46 connected to the parent bag 30 through a sealing member 44, a filter 48 for removing predetermined cells (for example, white blood cells and blood platelets) from the blood guided through the inlet-side tube 46, and an outlet-side tube 50 for guiding a residual blood component from which the predetermined cells have been removed through the filter 48, to the separation treatment portion 18.

The sealing member 44 has a similar configuration and function to the above-described sealing member 26. Note that the same applies to sealing members 62 and 72 described below. A clamp 52 that blocks and releases a flow path of the outlet-side tube 50 is provided in the middle portion of the outlet-side tube 50.

The separation treatment portion 18 includes a first child bag (first bag) 54 that houses (accumulates) the blood component guided through the outlet-side tube 50, a second child bag (second bag) 56 that houses and stores a supernatant component obtained through centrifugal separation of the blood component in the first child bag 54, a liquid medicine bag (third bag) 58 that houses a red blood cell preservation solution, and a transfer line 60 connected to the first child bag 54, the second child bag 56, and the liquid medicine bag 58.

The first child bag 54 functions as a bag for housing (accumulating) the residual blood component from which the predetermined cells have been removed through the filter 48, and as a bag for storing a sediment component (packed red blood cells) obtained through centrifugal separation of the blood component.

The transfer line 60 includes a first tube 64 connected to the first child bag 54 through a breakable sealing member 62, a Y-shaped branch connector 66 connected to the other end of the first tube 64, a second tube 68 that connects the branch connector 66 and the second child bag 56, a third tube 70 that connects the branch connector 66 and the liquid medicine bag 58, and a breakable sealing member 72 provided at the other end of the third tube 70.

Next, the initial flow blood bag 10 according to the present embodiment will be described. As illustrated in Fig. 2A, the initial flow blood bag 10 includes a bag body 80 configured in a symmetrical manner. The bag body 80 is configured such that flexible sheets made of a soft resin such as polyvinyl chloride or polyolefin are layered, and a peripheral sealed portion 81 thereof is sealed (thermally sealed or high-frequency sealed) or glued, in a bag-shaped manner.

Further, the bag body 80 is configured to have a rectangular shape in plan view, and includes a blood inflow portion 82, a first housing portion 84 as an air-housing portion provided with the blood inflow portion 82, a communication path 90 communicating into the first housing portion 84 and configured from a pair of partitions 86 and 88, a second housing portion 92 as an initial flow blood-housing portion communicating into the communication path 90, and a blood outflow portion 94 provided in the second housing portion 92.

The blood inflow portion 82 is connected to the other end of the branch tube 32 in a state of being provided in an approximately center in one end portion of the bag body 80 in a width direction. Note that the blood inflow portion 82 is open to a wall surface that configures the first housing portion 84.

The first housing portion 84 is arranged side by side with the second housing portion 92 along the longitudinal direction of the bag body 80. Further, the first housing portion 84 is set to have a size that can absorb variation of the amount of air in a blood-collecting line (the first blood-collecting tube 22, the branch connector 24, and the branch tube 32) from the blood-collecting needle 20 to the blood inflow portion 82, and is formed into a rectangular shape long in a short-length direction of the bag body 80 in plan view. Accordingly, the air in the blood-collecting line can be reliably housed in the first housing portion 84, and an increase in length of the bag body 80 can be suppressed.

The partitions 86 and 88 are used to partition an inside of the bag body 80 into the first housing portion 84 and the second housing portion 92. The partitions 86 and 88 extend in the width direction of the bag body 80, and end surfaces thereof face each other. That is, the end surfaces of the partitions 86 and 88 configure a part of the communication path 90.

The communication path 90 also functions as an excess initial flow blood accumulation portion that accumulates the initial flow blood that exceeds the capacity of the second housing portion 92 (referred to as excess initial flow blood). Further, the communication path 90 is positioned in an approximately center of the bag body 80 in the width direction. That is, an opening of the communication path 90 at the side of the first housing portion 84 faces an opening of the blood inflow portion 82. Therefore, the initial flow blood flowing in from the blood inflow portion 82 to the first housing portion 84 can be easily guided to the communication path 90.

The width of the communication path 90 is formed narrower than the first housing portion 84 and the second housing portion 92. Accordingly, an influence of inflation of the first housing portion 84 by the air on inflation of the second housing portion 92 can be made small. Further, a rate of change of the liquid level of the excess initial flow blood in the communication path 90 can be made larger than a rate of change of the liquid level of the initial flow blood in the second housing portion 92. Therefore, fine adjustment of the collection amount of the excess initial flow blood can be easily performed.

A width dimension L1 of the communication path 90 is smaller than a width dimension L2 of the second housing portion 92, and is favorably set to fall within a range of 30 to 60% of the width dimension L2, for example. In this case, the influence of inflation of the first housing portion 84 by the air on inflation of the second housing portion 92 can be adequately made small, and the initial flow blood flowing in from the blood inflow portion 82 can be smoothly guided to the second housing portion 92.

Note that length dimensions (a dimension along the longitudinal direction of the bag body 80, thickness dimensions of the partitions 86 and 88) of the communication path 90 can be arbitrarily set. A collectable amount of the excess initial flow blood can be easily adjusted by change of the length dimensions. In the present embodiment, the collectable amount of the excess initial flow blood is set to a capacity that is about 10% of the capacity of the second housing portion 92.

The second housing portion 92 is set to have a size that can house a predetermined amount of the initial flow blood (which is a necessary amount of the initial flow blood, and is 25 ml, for example). The blood outflow portion 94 is provided in an approximately center in the other end portion of the bag body 80 in the width direction. Further, the blood sampling holder 40 is attached to the blood outflow portion 94.

By the way, a piece of skin, skin indigenous bacteria, or the like may be mixed in the initial flow blood that flows out immediately after the blood-collecting needle 20 is inserted into the donor. Therefore, in the blood bag system 12 of the present embodiment, the initial flow blood is collected as the testing blood for blood transfusion in a preliminary step where the blood transfusion blood (whole blood) is collected from the donor.

In the present embodiment, when the initial flow blood is collected, the initial flow blood bag 10 is arranged to allow the initial flow blood to flow into the bag body 80 from a vertically upper side (hanging down arrangement, arranged as illustrated in Fig. 2A).

In collecting the initial flow blood, after the sealing member 26 is set to an initial state (blocked state) and the clamp 38 is set to a release state, the blood-collecting needle 20 is inserted into the donor. Then, the initial flow blood flowing out from the donor is guided to the first blood-collecting tube 22 through the blood-collecting needle 20, and the air in the blood-collecting line (the first blood-collecting tube 22, the branch connector 24, and the branch tube 32) flows from the blood inflow portion 82 into the first housing portion 84, and the first housing portion 84 is inflated.

Then, the initial flow blood that has flown into the first blood-collecting tube 22 is allowed to flow from the blood inflow portion 82 into the first housing portion 84 through the branch connector 24 and the branch tube 32, and is guided to the second housing portion 92 through the communication path 90. At this time, the liquid level of the initial flow blood in the second housing portion 92 rises, and the air in the second housing portion 92 is pressed by the initial flow blood and is discharged into the first housing portion 84 through the communication path 90.

Following that, at a point of time when the liquid level of the initial flow blood has reached a predetermined position (for example, a boundary portion between the second housing portion 92 and the communication path 90), the clamp 38 is closed, and the collection of the initial flow blood to the initial flow blood bag 10 is stopped (see Fig. 2B). Here, for example, to collect a larger amount of the initial flow blood than the capacity of the second housing portion 92 (necessary collection amount), the inflow of the initial flow blood to the bag body 80 is continued and the excess initial flow blood is accumulated in the communication path 90, and the clamp 38 may be closed at a point of time when the liquid level of the excess initial flow blood in the communication path 90 has reached a predetermined position.

In a state where the collection of the initial flow blood to the initial flow blood bag 10 is completed, the air in the second housing portion 92 is discharged to the first housing portion 84, and only the initial flow blood is housed in the second housing portion 92. That is, all of the air (approximately all of the air) in the initial flow blood bag 10 is housed in the first housing portion 84, and only the initial flow blood is housed in the second housing portion 92.

Since the communication path 90 is configured from the partitions 86 and 88, the influence of inflation of the first housing portion 84 by the air on inflation of the second housing portion 92 is small. Therefore, a fixed amount of the initial flow blood is collected to the initial flow blood bag 10.

Further, even if the amount of air that flows into the bag body 80 varies depending on the specification of the blood bag system 12 or a sterilization method, the variation can be absorbed in the first housing portion 84. Therefore, the air in the first housing portion 84 cannot flow into the second housing portion 92.

When the collection of the initial flow blood to the initial flow blood bag 10 is completed, the blood sampling tube 42 is mounted to the blood sampling holder 40, so that the initial flow blood in the bag body 80 is transferred to the blood sampling tube 42 in the depressurized state (vacuum state).

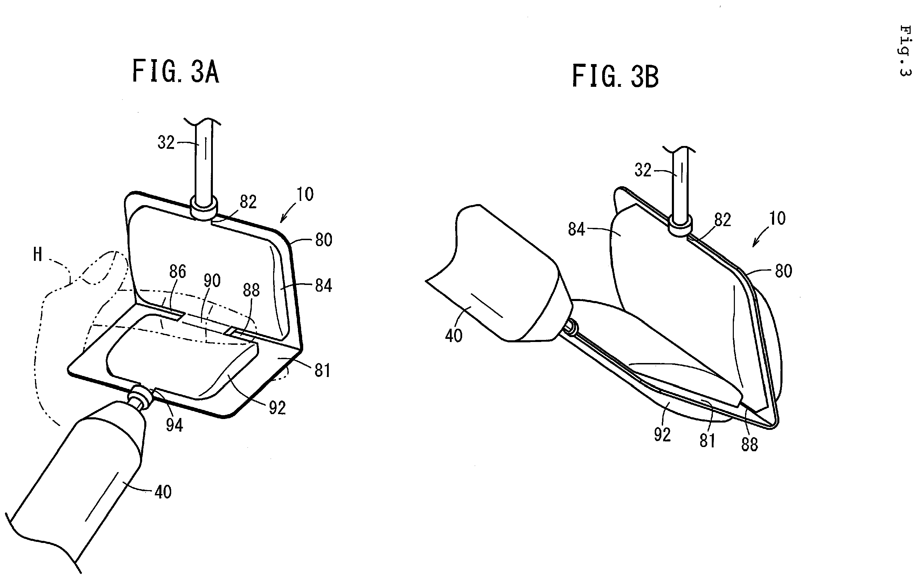

At this time, for example, as illustrated in Fig. 3A, if an opening (outer opening) of the blood outflow portion 94 at the side of the blood sampling holder 40 is directed in an approximately horizontal direction, in a state where a portion of the communication path 90 in the bag body 80 is pressed by fingers H and the portion is bent, and the flow path of the communication path 90 is blocked, the operator can perform the transfer work of the initial flow blood to the blood sampling tube 42 in a comfortable position without bending over, and inflow of the air discharged from the second housing portion 92 to the first housing portion 84, into the second housing portion 92 can be prevented. Accordingly, when the initial flow blood in the initial flow blood bag 10 is transferred to the blood sampling tube 42, the burden of the operator can be reduced, and the inflow of air to the blood sampling tube 42 can be suppressed.

Further, for example, as illustrated in Fig. 3B, when the portion of the communication path 90 in the bag body 80 is bent in an acute angle, the flow path of the communication path 90 can be blocked without pressing the portion with the fingers H, and the outer opening of the blood outflow portion 94 can be upwardly directed. In this case, an effect similar to the case of Fig. 3A can be exhibited.

When the collection of the initial flow blood ends, the breaking operation is performed with respect to the sealing member 26, and the flow path of the second blood-collecting tube 28 is opened, so that the predetermined amount of blood (whole blood) is collected in the parent bag 30. Following that, the white blood cells and the blood platelets of the whole blood in the parent bag 30 are removed through the filter 48, and the residual blood component is transferred to the first child bag 54. Then, the blood components in the first child bag 54 are separated into two components of the blood plasma and the packed red blood cells, using a centrifugal transfer device (not illustrated), and the blood plasma and the packed red blood cells are housed and stored in the different bags (the first child bag 54 and the second child bag 56).

According to the present embodiment, the communication path 90 is configured from the pair of partitions 86 and 88, which partitions the inside of the bag body 80 into the first housing portion 84 provided with the blood inflow portion 82 and the second housing portion 92 provided with the blood outflow portion 94. Therefore, the air in the second housing portion 92 can be discharged to the first housing portion 84 and only the initial flow blood can be housed in the second housing portion 92, and the influence of inflation of the first housing portion 84 by the air on inflation of the second housing portion 92 can be made small. Accordingly, a fixed amount of the initial flow blood can be collected to the initial flow blood bag 10.

Further, the portion of the communication path 90 in the bag body 80 is bent in a state where the air in the second housing portion 92 is discharged to the first housing portion 84, whereby the inflow of the air to the second housing portion 92 can be prevented. Accordingly, even if the blood sampling holder 40 and the blood sampling tube 42 connected to the blood outflow portion 94 are arranged at positions where the operator can easily perform the operation, the air cannot flow into the blood sampling tube 42. Therefore, the burden of the operator can be reduced, and the inflow of the air to the blood sampling tube 42 can be suppressed.

In the present embodiment, the pair of partitions 86 and 88 is provided to face each other along the width direction of the communication path 90. Therefore, the inflow of the initial flow blood housed in the second housing portion 92 to the first housing portion 84 can be favorably suppressed by the pair of partitions 86 and 88.

In the present embodiment, the initial flow blood may be collected in a state where the initial flow blood bag 10 is arranged such that the initial flow blood flows into the bag body 80 from an approximately horizontal direction (horizontal arrangement), depending on the structure of the blood-collecting bed, handling of the blood-collecting line, and the like.

In such a case, the second housing portion 92 is arranged vertically below the first housing portion 84 during collection of the initial flow blood, so that the air in the second housing portion 92 is discharged to the first housing portion 84, and only the blood can be housed in the second housing portion 92. Therefore, a fixed amount of blood can be collected to the initial flow blood bag 10.

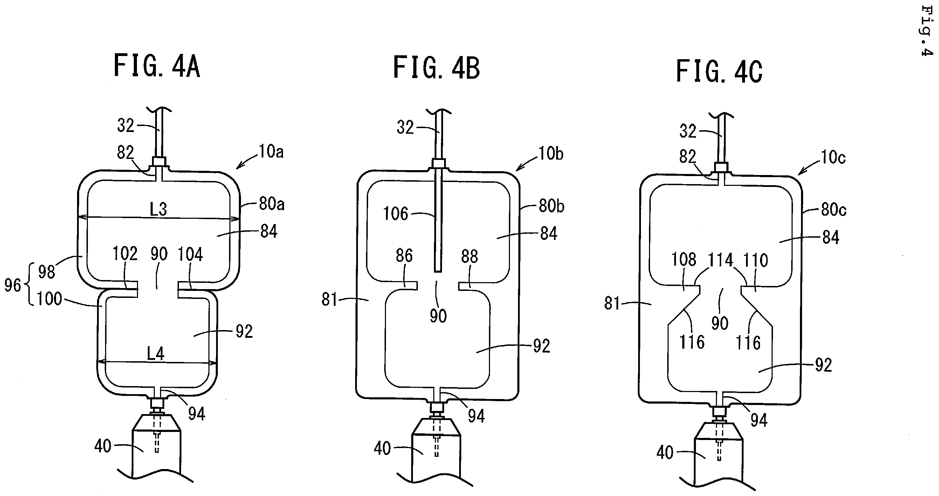

In the present embodiment, in the blood bag system 12, an initial flow blood bag 10a according to a first modification illustrated in Fig. 4A may be employed in place of the initial flow blood bag 10. Note that, in the first modification, a configuration common to the initial flow blood bag 10 is denoted with the same reference sign, and overlapping description is omitted. The same applies to initial flow blood bags 10b to 10e according to second to fifth modifications described below.

As illustrated in Fig. 4A, in the initial flow blood bag 10a, a configuration of a sealed portion 96 of a bag body 80a is different from the configuration of the sealed portion 81 of the bag body 80. The sealed portion 96 includes a first sealed part 98 in a periphery of a first housing portion 84, and a second sealed part 100 in a periphery of a second housing portion 92 formed independently of the first sealed part 98. That is, the first sealed part 98 and the second sealed part 100 are separately configured.

Further, an external dimension L4 of the second sealed part 100 along a width direction is set smaller than an external dimension L3 of the first sealed part 98 along the width direction. In this case, the second housing portion 92 and the second sealed part 100 can be easily held by fingers of a person, and thus the direction of an outer opening of a blood outflow portion 94 can be more easily changed.

In the initial flow blood bag 10a according to the present modification, the first sealed part 98 and the second sealed part 100 are fixed so that a communication path 90 is formed. That is, in such a bag body 80a, a pair of partitions 102 and 104 is formed in a fixed portion of the first sealed part 98 and the second sealed part 100.

According to the present modification, the first sealed part 98 in the periphery of the first housing portion 84 and the second sealed part 100 in the periphery of the second housing portion 92 are independently formed. Therefore, an influence of inflation of the first housing portion 84 by air on inflation of the second housing portion 92 can be made smaller.

Further, in the present embodiment, in the blood bag system 12, the initial flow blood bag 10b according to the second modification illustrated in Fig. 4B may be employed in place of the initial flow blood bag 10.

As illustrated in Fig. 4B, in the initial flow blood bag 10b, a configuration of a blood inflow portion 106 of a bag body 80b is different from the blood inflow portion 82 of the bag body 80. The blood inflow portion 106 extends toward a communication path 90 in a state of being configured in a tube-shaped manner, and one end thereof is connected to a branch tube 32, and the other end is positioned inside a first housing portion 84. That is, in the present modification, an opening of the blood inflow portion 106 is positioned inside the first housing portion 84.

According to the present modification, a distance between the opening of the blood inflow portion 106 and the communication path 90 can be made short. Therefore, initial flow blood flowing in from the blood inflow portion 106 can be more reliably guided to a second housing portion 92. That is, direct accumulation of the initial flow blood flowing in from the blood inflow portion 106, in the first housing portion 84 can be suppressed.

Further, in the present embodiment, in the blood bag system 12, the initial flow blood bag 10c according to the third modification illustrated in Fig. 4C may be employed in place of the initial flow blood bag 10.

As illustrated in Fig. 4C, in the initial flow blood bag 10c, configurations of partitions 108 and 110 of a bag body 80c are different from the configurations of the partitions 86 and 88 of the bag body 80. Flat portions 114 continuing to side wall surfaces that configure a first housing portion 84 in an approximately right angle manner are formed on wall surfaces of the partitions 108 and 110, which configure the first housing portion 84. Further, inclined portions 116 close to a communication path 90 toward the first housing portion 84 are formed on wall surfaces of the partitions 108 and 110, which configure a second housing portion 92.

According to the present modification, the flat portions 114 formed on the wall surfaces of the partitions 108 and 110, which configure the first housing portion 84 continue to the side wall surfaces that configure the first housing portion 84 in an approximately right angle manner. Therefore, inflow of air in the first housing portion 84 to the second housing portion 92 through the communication path 90 can be favorably suppressed.

Further, the inclined portions 116 formed on the wall surfaces of the partitions 108 and 110, which configure the second housing portion 92 are close to the communication path 90 toward the first housing portion 84. Therefore, the air in the second housing portion 92 can be smoothly guided to the first housing portion 84 through the inclined portions 116.

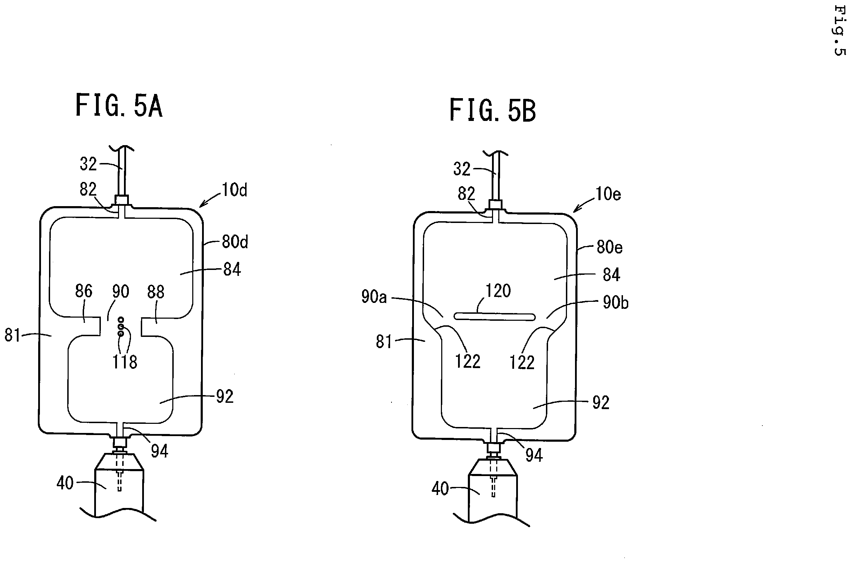

Further, in the present embodiment, in the blood bag system 12, the initial flow blood bag 10d according to the fourth modification illustrated in Fig. 5A may be employed in place of the initial flow blood bag 10.

As illustrated in Fig. 5A, in the initial flow blood bag 10d, a plurality of marks (display means) 118 with which the amount of initial flow blood housed in a bag body 80d can be confirmed is attached to a wall portion that configures a communication path 90 of the bag body 80d. In this case, the collection amount of the initial flow blood in the initial flow blood bag 10d can be easily confirmed. Therefore, adjustment of the collection amount of the initial flow blood can be easily performed.

Further, in the present embodiment, in the blood bag system 12, the initial flow blood bag 10e according to the fifth modification illustrated in Fig. 5B may be employed in place of the initial flow blood bag 10.

As illustrated in Fig. 5B, in the initial flow blood bag 10e, a pair of communication paths 90a and 90b are formed by a partition 120 extending along a width direction of a bag body 80e in a state of being positioned in an approximately center of the bag body 80e in the width direction. That is, the pair of communication paths 90a and 90b is provided at both end sides of the partition 120.

Further, guiding portions 122 inclined in a direction into which the width of a second housing portion 92 is enlarged toward a first housing portion 84 are formed in portions of a sealed portion 81, which configure one end side (a side where the first housing portion 84 is positioned) of the second housing portion 92.

According to the present modification, the partition 120 extends along the width direction of the bag body 80e in a state of being positioned in an approximately center of the bag body 80e in the width direction. Therefore, an influence of inflation of the first housing portion 84 by air on inflation of the second housing portion 92 can be made smaller.

Further, the pair of communication paths 90a and 90b is provided at both end sides of the partition 120. Therefore, the initial flow blood flowing in from a blood inflow portion 82 can be smoothly guided to the second housing portion 92, compared with a case where the communication path 90a or the communication path 90b is provided at only one end portion side of the partition 120.

Further, the pair of guiding portions 122 is formed in the portions of the sealed portion 81, which configure the one end side of the second housing portion 92. Therefore, air in the second housing portion 92 can be smoothly guided to the first housing portion 84 through the guiding portions 122.

Favorable embodiments about the present invention have been described. However, the present invention is not limited to the embodiments, and various alternations can be made.

For example, the blood bag according to the present invention is not limited to the initial flow blood bag, and may be a blood bag used for other uses.

## Claims

1. A blood bag (10, 10a to 10e) including a bag body (80, 80a to 80e) formed of a soft material in a bag-shaped manner, and being able to house blood,
the bag body (80, 80a to 80e) comprising:
a first housing portion (84) provided with a blood inflow portion (82, 106);
a second housing portion (92) provided with a blood outflow portion (94); and
a communication path (90, 90a, 90b) configured from a partition (86, 88, 102, 104, 108, 110, 120) that partitions an inside of the bag body (80, 80a to 80e) into the first housing portion (84) and the second housing portion (92), and allowing the first housing portion (84) and the second housing portion (92) to communicate with each other, **characterized in that**
when an outer opening of the blood outflow portion (94) at the side of a blood sampling holder (40) is directed in an approximately horizontal direction, it is configured to be in a state where a portion of the communication path (90) in the bag body (80) may be pressed in order to bend the blood outflow portion (94) and thus block the flow path of the communication path (90) to prevent inflow of air discharged from the second housing portion (92) to the first housing portion (84), into the second housing portion (92), whereas when the portion of the communication path (90) in the bag body (80) is bent in an acute angle, it is constructed to block the flow path of the communication path (90) without pressing the outflow portion (94), and the outer opening of the blood outflow portion (94) can be upwardly directed.

2. The blood bag (10, 10a to 10d) according to claim 1, wherein
an opening of the blood inflow portion (82, 106) faces an opening in the communication path (90) at a side of the first housing portion (84).

3. The blood bag (10, 10a to 10d) according to claim 1, wherein
a pair of the partitions (86, 88, 102, 104, 108, 110) is provided to face each other along a width direction of the communication path (90).

4. The blood bag (10a) according to claim 1, wherein
the bag body (80a) is configured such that soft sheet materials are layered, and a sealed portion (96) in a periphery of the seat materials is sealed or glued, and
the sealed portion (96) includes a first sealed part (98) in a periphery of the first housing portion (84), and a second sealed part (100) in a periphery of the second housing portion (92) and formed independently of the first sealed part (98).

5. The blood bag (10a) according to claim 4, wherein
an external dimension (L4) along a width direction of the second sealed part (100) is set smaller than an external dimension (L3) along a width direction of the first sealed part (98).

6. The blood bag (10b) according to claim 1, wherein
the blood inflow portion (106) extends toward the communication path (90) in a state of being configured in a tube-shaped manner, and
an opening of the blood inflow portion (106) is positioned inside the first housing portion (84).

7. The blood bag (10c) according to claim 1, wherein
an inclined portion (116) close to the communication path (90) toward the first housing portion (84) is formed on a wall surface of the partition (108, 110), the wall surface configuring the second housing portion (92).

8. The blood bag (10c) according to claim 7, wherein
a flat portion (114) continuing to a side wall surface that configures the first housing portion (84), in an approximately right angle manner, is formed on a wall surface of the partition (108, 110), the wall surface configuring the first housing portion (84).

9. The blood bag (10d) according to claim 1, wherein
a display means (118) with which an amount of blood housed in the bag body (80d) is able to be confirmed is attached to a wall portion that configures the communication path (90).

10. The blood bag (10e) according to claim 1, wherein
the partition (120) extends along a width direction of the bag body (80e) in a state of being positioned in an approximately center of the bag body (80e) in the width direction, and
a pair of the communication paths (90a, 90b) is provided at both end sides of the bag body (80e).

11. The blood bag (10, 10a to 10e) according to claim 1, wherein
the blood housed in the bag body (80, 80a to 80e) is initial flow blood.

12. The blood bag (10, 10a to 10e) according to claim 1, wherein
all air in the blood bag (10, 10a to 10e) is housed in the first housing portion (84), and
only the blood is housed in the second housing portion (92).

13. A blood bag system (12) comprising:
a blood bag (10, 10a to 10e) for housing initial flow blood;
a blood-collecting bag (30) for housing blood after the initial flow blood is removed; and
a separation treatment portion (18) configured to divide the blood housed in the blood-collecting bag (30) into a plurality of blood components, and to house the respective components in different bags (54, 56), wherein
the blood bag (10, 10a to 10e) is a blood bag (10, 10a to 10e) according to any one of claims 1 to 12.

## Patentansprüche

1. Blutbeutel (10, 10a bis 10e), der einen Beutelkörper (80, 80a bis 80e) umfasst, welcher aus einem weichen Material in einer beutelförmigen Weise gebildet und in der Lage ist, Blut aufzunehmen,
wobei der Beutelkörper (80, 80a bis 80e) umfasst:
einen ersten Gehäuseabschnitt (84), der mit einem Bluteinströmabschnitt (82, 106) versehen ist;
einen zweiten Gehäuseabschnitt (92), der mit einem Blutausströmabschnitt (94) versehen ist; und
einen Kommunikationsweg (90, 90a, 90b), der von einer Trennwand (86, 88, 102, 104, 108, 110, 120) konfiguriert ist, die eine Innenseite des Beutelkörpers (80, 80a bis 80e) in den ersten Gehäuseabschnitt (84) und den zweiten Gehäuseabschnitt (92) unterteilt und es dem ersten Gehäuseabschnitt (84) und dem zweiten Gehäuseabschnitt (92) ermöglicht, miteinander zu kommunizieren, **dadurch gekennzeichnet, dass**
wenn eine äußere Öffnung des Blutausströmabschnitts (94) an der Seite eines Blutprobenentnahmehalters (40) in eine annähernd horizontale Richtung gerichtet ist, sie so konfiguriert ist, dass sie in einem Zustand ist, in dem ein Abschnitt des Kommunikationsweges (90) in dem Beutelkörper (80) gedrückt werden kann, um den Blutausströmabschnitt (94) zu biegen und hierdurch den Strömungsweg des Kommunikationsweges (90) zu blockieren, um den Einstrom von Luft, die von dem zweiten Gehäuseabschnitt (92) zu dem ersten Gehäuseabschnitt (84) abgegeben wird, in den zweiten Gehäuseabschnitt (92) zu verhindern, wobei hingegen, wenn der Abschnitt des Kommunikationsweges (90) in dem Beutelkörper (80) in einem spitzen Winkel gebogen ist, er so konstruiert ist, dass er den Strömungsweg des Kommunikationsweges (90) blockiert, ohne den Ausströmabschnitt (94) zu drücken, und die äußere Öffnung des Blutausströmabschnittes (94) nach oben gerichtet werden kann.

2. Blutbeutel (10, 10a bis 10d) nach Anspruch 1, wobei
eine Öffnung des Bluteinströmabschnitts (82, 106) einer Öffnung in dem Kommunikationsweg (90) an einer Seite des ersten Gehäuseabschnitts (84) zugewandt ist.

3. Blutbeutel (10, 10,10a bis 10d) nach Anspruch 1, wobei
ein Paar der Trennwände (86, 88, 102, 104, 108, 110) vorgesehen ist, dass sie einander entlang einer Breitenrichtung des Kommunikationsweges (90) gegenüberliegen.

4. Blutbeutel (10a) nach Anspruch 1, wobei
der Beutelkörper (80a) derart konfiguriert ist, dass weiche Folienmaterialien geschichtet sind, und ein abgedichteter Abschnitt (96) in einem Umfang der Sitzmaterialien abgedichtet oder zusammengeklebt ist, und
der abgedichtete Abschnitt (96) einen ersten abgedichteten Teil (98) in einem Umfang des ersten Gehäuseabschnitts (84) und einen zweiten abgedichteten Teil (100) in einem Umfang des zweiten Gehäuseabschnitts (92) aufweist und unabhängig von dem ersten abgedichteten Teil (98) ausgebildet ist.

5. Blutbeutel (10a) nach Anspruch 4, wobei
eine Außenabmessung (L4) entlang einer Breitenrichtung des zweiten abgedichteten Teils (100) kleiner als eine Außenabmessung (L3) entlang einer Breitenrichtung des ersten abgedichteten Teils (98) eingestellt ist.

6. Blutbeutel (10b) nach Anspruch 1, wobei
der Bluteinströmabschnitt (106) sich in einem Zustand, in dem er in einer röhrenförmigen Weise ausgebildet ist, in Richtung des Kommunikationsweges (90) erstreckt, und
eine Öffnung des Bluteinströmabschnitts (106) innerhalb des ersten Gehäuseabschnitts (84) angeordnet ist.

7. Blutbeutel (10c) nach Anspruch 1, wobei
ein abgeschrägter Abschnitt (116) in der Nähe des Kommunikationsweges (90) in Richtung des ersten Gehäuseabschnitts (84) auf einer Wandfläche der Trennwand (108, 110) ausgebildet ist, wobei die Wandfläche den zweiten Gehäuseabschnitt (92) konfiguriert.

8. Blutbeutel (10c) nach Anspruch 7, wobei
ein flacher Abschnitt (114), der sich zu einer Seitenwandfläche fortsetzt, die den ersten Gehäuseabschnitt (84) in einer annähernd rechtwinkligen Weise konfiguriert, auf einer Wandfläche der Trennwand (108, 110) ausgebildet ist, wobei die Wandfläche den ersten Gehäuseabschnitt (84) konfiguriert.

9. Blutbeutel (10d) nach Anspruch 1, wobei
eine Anzeigeeinrichtung (118), mit der eine in dem Beutelkörper (80d) aufgenommene Blutmenge bestätigt werden kann, an einem Wandabschnitt angebracht ist, der den Kommunikationsweg (90) konfiguriert.

10. Blutbeutel (10e) nach Anspruch 1, wobei
die Trennwand (120) sich entlang einer Breitenrichtung des Beutelkörpers (80e) in einem Zustand erstreckt, in dem sie in der Breitenrichtung annähernd in der Mitte des Beutelkörpers (80e) positioniert ist, und
ein Paar der Kommunikationswege (90a, 90b) an beiden Endseiten des Beutelkörpers (80e) vorgesehen ist.

11. Blutbeutel (10, 10a bis 10e) nach Anspruch 1, wobei
das im Beutelkörper (80, 80a bis 80e) aufgenommene Blut am Anfang fließendes Blut ist.

12. Blutbeutel (10, 10a bis 10e) nach Anspruch 1, wobei
die gesamte Luft in dem Blutbeutel (10, 10a bis 10e) in dem ersten Gehäuseabschnitt (84) aufgenommen ist und nur das Blut in dem zweiten Gehäuseabschnitt (92) aufgenommen ist.

13. Blutbeutelsystem (12), umfassend:
einen Blutbeutel (10, 10a bis 10e) zum Aufnehmen des am Anfang fließenden Blutes;
einen Blutsammelbeutel (30), der Blut aufnimmt, nachdem das am Anfang fließende Blut entfernt ist; und
einen Trennungsbehandlungsabschnitt (18), der so konfiguriert ist, dass er das in dem Blutsammelbeutel (30) aufgenommene Blut in eine Mehrzahl von Blutkomponenten unterteilt und die jeweiligen Komponenten in verschiedenen Beuteln (54, 56) aufnimmt, wobei
der Blutbeutel (10, 10a bis 10e) ein Blutbeutel (10, 10a bis 10e) nach einem der Ansprüche 1 bis 12 ist.

## Revendications

1. Poche de sang (10, 10a à 10e) comprenant un corps de poche (80, 80a à 80e) formé d'un matériau souple en forme de poche, et pouvant contenir du sang,
le corps de poche (80, 80a à 80e) comprenant :
une première partie de stockage (84) munie d'une partie d'entrée de sang (82, 106) ;
une seconde partie de stockage (92) munie d'une partie de sortie de sang (94) ; et
un trajet de communication (90, 90a, 90b) conçu à partir d'une cloison (86, 88, 102, 104, 108, 110, 120) qui divise l'intérieur du corps de poche (80, 80a à 80e) en la première partie de stockage (84) et la seconde partie de stockage (92), et permettant à la première partie de stockage (84) et à la seconde partie de stockage (92) de communiquer l'une avec l'autre, **caractérisée en ce que**
lorsqu'une ouverture extérieure de la partie de sortie de sang (94) au niveau du côté d'un support de prélèvement de sang (40) est dirigée dans une direction approximativement horizontale, elle est conçue pour être dans un état où une partie du trajet de communication (90) dans le corps de poche (80) peut être pressée afin de faire fléchir la partie de sortie de sang (94) et de bloquer ainsi le circuit d'écoulement du trajet de communication (90) pour empêcher que l'air déchargé depuis la seconde partie de stockage (92) dans la première partie de stockage (84) n'entre dans la seconde partie de stockage (92), tandis que, lorsque la partie du trajet de communication (90) dans le corps de poche (80) est pliée selon un angle aigu, elle est conçue pour bloquer le circuit d'écoulement du trajet de communication (90) sans presser la partie de sortie de sang (94), et l'ouverture extérieure de la partie de sortie de sang (94) peut être dirigée vers le haut.

2. Poche de sang (10, 10a à 10d) selon la revendication 1, dans laquelle
une ouverture de la partie d'entrée de sang (82, 106) est tournée vers une ouverture du trajet de communication (90) au niveau d'un côté de la première partie de stockage (84).

3. Poche de sang (10, 10a à 10d) selon la revendication 1, dans laquelle
une paire de cloisons (86, 88, 102, 104, 108, 110) est prévue pour se faire face mutuellement dans le sens de la largeur du trajet de communication (90).

4. Poche de sang (10a) selon la revendication 1, dans laquelle
le corps de poche (80a) est conçu de telle sorte que des matériaux souples en forme de feuille sont disposés en couches, et une partie scellée (96) dans une périphérie des matériaux est scellée ou collée, et
la partie scellée (96) comprend une première partie scellée (98) dans une périphérie de la première partie de stockage (84), et une seconde partie scellée (100) dans une périphérie de la seconde partie de stockage (92) et formée indépendamment de la première partie scellée (98).

5. Poche de sang (10a) selon la revendication 4, dans laquelle
une dimension externe (L4) dans le sens de la largeur de la seconde partie scellée (100) est réglée pour être inférieure à une dimension externe (L3) dans le sens de la largeur de la première partie scellée (98).

6. Poche de sang (10b) selon la revendication 1, dans laquelle
la partie d'entrée de sang (106) s'étend vers le trajet de communication (90) dans un état où elle est en forme de tube, et
une ouverture de la partie d'entrée de sang (106) est positionnée à l'intérieur de la première partie de stockage (84).

7. Poche de sang (10c) selon la revendication 1, dans laquelle
une partie inclinée (116) proche du trajet de communication (90) vers la première partie de stockage (84) est formée sur une surface de paroi de la cloison (108, 110), la surface de paroi constituant la seconde partie de stockage (92).

8. Poche de sang (10c) selon la revendication 7, dans laquelle
une partie plate (114) qui s'étend jusqu'à une surface de paroi latérale qui constitue la première partie de stockage (84), approximativement à angle droit, est formée sur une surface de paroi de la cloison (108, 110), la surface de paroi constituant la première partie de stockage (84).

9. Poche de sang (10d) selon la revendication 1, dans laquelle
un moyen d'affichage (118) avec lequel une quantité de sang stocké dans le corps de poche (80d) peut être confirmée est fixé à une partie de paroi qui constitue le trajet de communication (90).

10. Poche de sang (10e) selon la revendication 1, dans laquelle
la cloison (120) s'étend dans le sens de la largeur du corps de poche (80e) dans un état où elle est placée approximativement au centre du corps de poche (80e) dans le sens de la largeur, et une paire de trajets de communication (90a, 90b) est prévue au niveau des deux côtés d'extrémité du corps de poche (80e).

11. Poche de sang (10, 10a à 10e) selon la revendication 1, dans laquelle le sang stocké dans le corps de poche (80, 80a à 80e) est du sang à écoulement initial.

12. Poche de sang (10, 10a à 10e) selon la revendication 1, dans laquelle tout l'air de la poche de sang (10, 10a à 10e) est stocké dans la première partie de stockage (84), et seul le sang est stocké dans la seconde partie de stockage (92).

13. Système de poche de sang (12) comprenant :
une poche de sang (10, 10a à 10e) destinée à stocker du sang à écoulement initial ;
une poche de collecte de sang (30) pour stocker le sang après que le sang à écoulement initial est retiré ; et
une partie de traitement de séparation (18) conçue pour diviser le sang stocké dans la poche de collecte de sang (30) en une pluralité de composants sanguins, et pour stocker les composants respectifs dans différentes poches (54, 56), où
la poche de sang (10, 10a à 10e) est une poche de sang (10, 10a à 10e) selon l'une quelconque des revendications 1 à 12.
